Europäisches Patentamt

European Patent Office

Office européen des brevets

(11) Publication number: **0 337 677**
**A2**

## (12) EUROPEAN PATENT APPLICATION

(21) Application number: **89303463.7**

(22) Date of filing: **07.04.89**

(51) Int. Cl.⁴: **B65D 51/16**

(30) Priority: **11.04.88 US 179764**
**21.12.88 US 287848**

(43) Date of publication of application:
**18.10.89 Bulletin 89/42**

(84) Designated Contracting States:
**DE FR GB IT**

(71) Applicant: **COSTAR CORPORATION**
**One Alewife Center**
**Cambridge Massachusetts 02140(US)**

(72) Inventor: **Eudailey, William**
**76 Grandville Lane**
**No. Andover Massachusetts 01845(US)**
Inventor: **Lyman, George**
**Rocky Point**
**Cape Porpoise Massachusetts 04014(US)**

(74) Representative: **Woodward, John Calvin et al**
**VENNER SHIPLEY & CO. 368 City Road**
**London EC1V 2QA(GB)**

㊹ **Vent cap.**

㊷ A vent cap for tissue culture flasks having an internally threaded cylindrical wall and an end wall. An annular seat is provided at the periphery of the inner surface of the end wall to seal against the rim of the flask, and a vent opening extends through the end wall radially inwardly of the seat. A hydrophobic membrane is secured to the inner surface of the end wall and covers the vent opening, which permits the exchange of gas through the opening while prohibiting the exchange of liquids, bacteria and contaminants through the opening.

EP 0 337 677 A2

## VENT CAP

This invention relates to a new and improved vent cap for tissue culture flasks and more particularly comprises a vent cap which eliminates the need for periodic "cracking" to permit gas exchange, maintains the inside of the flask sterile, prevents the escape of water vapour and sterilizes gas which escapes from the flask.

Conventional tissue culture flask caps maintain a sterile condition within the flasks by forming a seal about the rim of the flask neck. In order to permit an exchange of carbon dioxide and oxygen between the flask interior and the atmosphere, it is necessary to loosen the cap on the neck, which breaks the seal. While this so called "cracking" of the cap permits the required gas exchange, it also breaks the sterile seal of the flask, which may have very serious consequences. For example, with certain types of research, the opening of the flask by loosening the cover may result in the escape of dangerous bacteria or other material and expose those who handle the flask.

Another drawback of conventional flask covers results from the increased use of convection type incubators. The agitated atmosphere in that type of incubator increases the likelihood of contamination if the flask cover is unsealed.

Yet another problem encountered with conventional flask caps is the build up of pressure in the flasks which creates leaks. This condition is particularly troublesome when dangerous cultures are being grown.

The primary object of this invention is to provide a vent cap for tissue culture flasks which will maintain a sterile atmosphere within a flask while at the same time allowing gas exchange between the flask interior and its surroundings. Stated in another way, an important object of this invention is to provide a flask cap which need not be opened to permit gas exchange.

Another important object of the present invention is to provide a flask cap which keeps the inside of the flask isolated from the general environment and from anyone handling the flask while permitting the exchange of CO and oxygen through the cap.

To accomplish these and other objects, the flask cap of this invention is provided with an array of small openings in its end wall which are covered by a hydrophobic microporous membrane on the inside having a pore size small enough to prevent the passage of bacteria or contaminants and which nevertheless permits the exchange of gas through the openings. The hydrophobic membrane prevents water in the flask from leaking out. The microporous membrane will not become wetted and close down even if the water inside contacts the membrane.

The outer surface of the cap through which the openings extend is flat so that they may be readily closed by a strip of tape whenever it is desired to interrupt the exchange of gas between the atmosphere and the interior of the flask.

Another feature of this invention is the presence of a plenum chamber between the openings and the membrane. The plenum chamber allows the full area of the membrane to be utilized even if one or more of the openings is closed.

The cap of this invention has many advantages in addition to those described above. For example, the gas exchange properties of the membrane permit a common atmosphere to be created between the flask interior and the environment. Cultures are typically grown in flasks placed in "$CO_2$ air" incubators. This is done because there are ideal temperature and atmospheric requirements for optimal pH and growth conditions. The membrane ensures that the atmospheric conditions inside the flask are the same as in the incubator.

Another advantage is that a flask covered with the cap of this invention is not susceptible to contamination when placed in a convection type incubator where the atmosphere is agitated. The membrane allows only gas exchange and the flask remains sealed. Additionally, the vent cap of this invention prevents a build up of pressure in the flask, which therefore prevents the welds or other seals in the flask from being ruptured. This feature has particular application in flasks used to grow artificial skin. Those flasks may have special covers which may be opened to provide access to the flask interior, and the vent cap of this invention will prevent pressure from building up in the flask which could otherwise blow open those covers.

An additional feature of the present invention is that it prevents contaminated air from being sucked into a flask when the flask is removed from an incubator. When a conventional cap is used with the cap "cracked" and the flask is removed from an incubator, the flask cools down and a negative pressure may develop that will suck non-sterile air through the "cracked" cap into the flask.

These and other objects, features and advantages will be better understood and appreciated from the following detailed description read with reference to the accompanying drawings.

Figure 1 is a perspective view of a flask carrying a cap, constructed in accordance with this invention;

Figure 2 is a fragmentary cross-sectional view through the cap and flask neck shown in Figure 1; and

Figure 3 is an enlarged fragmentary cross-sectional view of the cap of Figures 1 and 2.

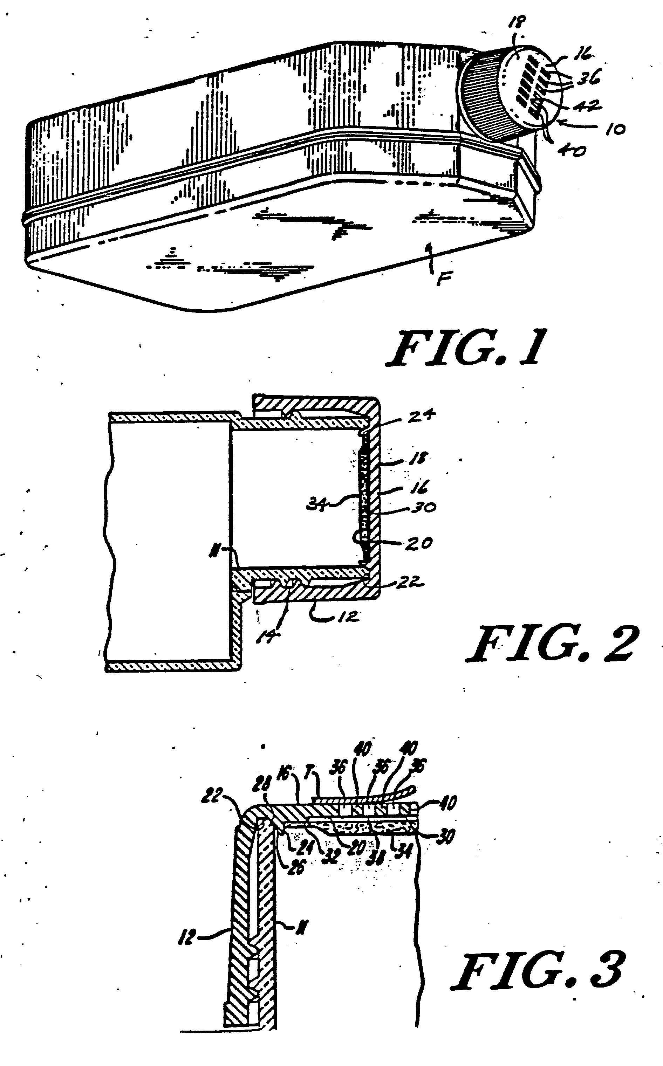

In Figure 1 a tissue culture flask F is shown carrying a vent cap 10 on the neck N of the flask. The cap 10 embodies the present invention.

Vent cap 10 includes a generally cylindrical side wall 12 internally threaded as suggested at 14 and an end or top wall 16 which essentially closes one end of the cylindrical wall 12. In the preferred form, the outer surface 18 of the end wall is flat for purposes which are described below.

The inner surface 20 of end wall 16 has an annular sealing seat 22 about its periphery defined at its inner edge by a rib 24 having an inclined outer surface 26 which is designed to seat against the inner edge 28 of the rim of the neck N of the flask to form a seal with it when the threaded cylindrical wall 12 is screwed tightly onto the neck. The configuration of the rib 26 enables the cap 10 to form a seal with the rim of the neck N of the flask without the use of a rubber gasket or other sealing material.

The circular area 30 of the end wall 16 within the rib 24 has a shoulder 32 to which the periphery of the hydrophobic microporous membrane 34 is heat sealed. The membrane 34 typically may be made of an acrylic copolymer and have a pore size of 0.2 microns. An example of such a membrane is manufactured by Gelman Corporation under the trademark VERSOPORE.

Inside shoulder 32 in the circular area 30 is an array of vent openings 36 which extend through the end wall 16 and which allow the inside of the flask to communicate with the atmosphere as limited by the hydrophobic membrane 34. A variety of different patterns may be established for the openings 36. Regardless of the pattern used, the individual openings should be relatively small and be separated by ribs which will support the portions of the membrane spanning the area 30 when pressure within the flask exceeds ambient pressure.

The main body of the membrane 34 which spans the array of vent openings 36 defines a plenum chamber 38 under the inner surface 20 of the end wall 16, that permits the individual openings to communicate with substantially the full surface of the membrane. This is desirable because it permits full use of the membrane even when one or more of the openings 36 becomes clogged.

In the pattern of the vent openings 36 shown, a number of parallel ribs 40 extend perpendicular to the central rib 42 to describe 12 separate openings 36 in the end wall. The ribs 40 and 42 will support the membrane 34 when pressure in the flask exceeds ambient pressure. The use of a hydrophobic membrane 34 enables the inside of the flask to be maintained in a sterile condition and further retains water vapour in the flask while allowing the exchange of carbon dioxide and oxygen between the flask interior and the atmosphere. The hydrophobic microporous membrane also sterilizes any gas which is emitted from the flask. The pore size of the membrane is small enough to prevent the passage of bacteria or other contaminants while allowing the free gas exchange described. The membrane also prevents water from leaking from the flask, and the pores will not close down even if the membrane becomes wetted as the result of tilting or other action, which would interfere with the gas exchange.

A very important advantage of the vent cap of this invention is its ability to contain the flask contents, that is, to isolate the inside of the flask F from the general environment. This is particularly important when dangerous research is being conducted. The vent cap will prohibit the escape of bacteria and other materials, and yet allows the gas exchange.

The cap has particular application with tissue culture flasks used in carbon dioxide/air incubators. The cap permits a common atmosphere inside the flask and in the incubator. In the past, this has only been accomplished by opening the normally sealed caps slightly to allow the gas exchange to occur.

It should also be appreciated that more and more incubators are of the convection type. The increased agitation of the incubator atmosphere increases the likelihood of contamination if the conventional method of "cracking" caps is used for venting purposes. The present invention prevents contamination as the hydrophobic membrane eliminates the need for "cracking" the cap.

As yet another advantage, the cap of the present invention prevents pressure from building up in the flask when the gases are generated in it. For example, if the flask is used for growing tissue, flask pressure builds up as the tissue culture generates $CO_2$ gas. The build up of pressure sometimes results in the breaking of the ultrasonic welds in the flasks causing leaking to occur.

The vent cap of the present invention has other advantages. It should be appreciated that it provides a seal for the flask without the use of liners, gaskets, or other materials. Because the membrane is confined to the area of the cap which is not in contact with the flask neck, the membrane is not subject to rubbing against the bottle and therefore will not accidentally be torn by it or otherwise lose its integrity. Furthermore, the flat outer surface 18 of the cap permits the user to close off all or a portion of the vent openings 36 with a piece of tape on the surface. This is suggested in Figure 3 by the tape T.

From the foregoing description, those skilled in the art will appreciate that numerous modifications may be made of this invention without departing from its spirit. Therefore, it is not intended that the scope of this invention be limited to the single embodiment illustrated and described. Rather, the scope of the invention is to be determined by the appended claims and their equivalents.

## Claims

1. A vent cap for a tissue culture flask comprising an internally threaded cylindrical wall, an end wall extending across one end of the cylindrical wall, an annular seat on the inside of the end wall facing the cylindrical wall for sealing against the rim of a flask, a vent opening in the end wall and disposed within the area enclosed by the annular seat, and a hydrophobic membrane secured to the inside of the end wall and covering the vent opening for permitting the exchange of gas through the vent opening while prohibiting the exchange of liquids, bacteria and contaminants through said opening.

2. A vent cap as defined in claim 1 wherein said seat is defined by a circular bead formed on the inside of the end wall and having a beveled surface for bearing against the rim of a flask.

3. A vent cap as defined in claims 1 or 2 wherein the outer surface of the end wall is smooth so that a strip of tape may be applied to the surface to close or reduce the effective size of the vent opening.

4. A vent cap as claimed in any of claims 1-3 wherein the vent opening comprises a plurality of closely spaced holes extending through the end wall.

5. A vent cap as claimed in any of claims 1-4 wherein a shoulder is provided on the inside of the end wall about the closely spaced holes and within the annular seat against which the membrane is sealed to the end wall.

6. A vent cap as defined in claim 1 wherein the hydrophobic membrane is confined to the area radially inwardly of the annular seat.

7. A vent cap as defined in claim 3 wherein the hydrophobic membrane is confined to the area radially inwardly of the circular bead.

8. A vent cap as defined in claim 1 wherein a plenum chamber is provided between the membrane and the end wall placing all parts of the opening in communication with a substantial portion of the membrane.

9. A vent cap as defined in claim 4 wherein a plenum chamber is provided between the membrane and the end wall covering the area of all the holes which comprise the opening for placing each hole in communication with the membrane.

10. A vent cap for a tissue culture flask comprising an internally threaded cylindrical wall, an end wall extending across one end of the cylindrical wall, an annular seat on the inside of the end wall for sealing against the rim of a flask, a vent opening in the end wall and disposed within the area enclosed by the annular seat, and a hydrophobic microporous membrane secured to the end wall and covering the vent opening for permitting the exchange of gas through the vent opening while prohibiting the exchange of liquids, bacteria and contaminants through said opening.

11. A vent cap for tissue culture flasks as defined in claim 10 wherein the membrane is secured to the inside of the end wall.

12. A vent cap for tissue culture flasks comprising an internally threaded cylindrical wall, an end wall extending across one end of the cylindrical wall, an annular seat on the inside of the end wall for sealing against the rim of a flask, a vent opening in the end wall and disposed within the area enclosed by the annular seat, and a microporous membrane secured to the end wall and covering the vent opening for permitting the exchange of gas through the vent opening while prohibiting the exchange of bacteria and contaminants through said opening.

*FIG. 1*

*FIG. 2*

*FIG. 3*